(19) Europäisches Patentamt
European Patent Office
Office européen des brevets

(11) EP 3 488 236 B1

(12) EUROPEAN PATENT SPECIFICATION

(45) Date of publication and mention
of the grant of the patent:
08.07.2020 Bulletin 2020/28

(51) Int Cl.:
$G01N\ 27/327$ (2006.01)    $G01N\ 33/543$ (2006.01)
$G01N\ 33/94$ (2006.01)    $C01B\ 32/184$ (2017.01)

(21) Application number: 17748945.7

(22) Date of filing: 18.07.2017

(86) International application number:
PCT/IB2017/054337

(87) International publication number:
WO 2018/015884 (25.01.2018 Gazette 2018/04)

(54) **ON-CHIP GRAPHENE ELECTRODE, METHODS OF MAKING, AND METHODS OF USE**

ON-CHIP-GRAPHENELEKTRODE, VERFAHREN ZUR HERSTELLUNG UND VERFAHREN ZUR VERWENDUNG

ÉLECTRODE DE GRAPHÈNE SUR PUCE, PROCÉDÉS DE PRODUCTION ET PROCÉDÉS D'UTILISATION

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 21.07.2016 US 201662364917 P

(43) Date of publication of application:
29.05.2019 Bulletin 2019/22

(73) Proprietors:
• King Abdullah University Of Science And Technology
Thuwal 23955-6900 (SA)
• Universität Regensburg
93053 Regensburg (DE)

(72) Inventors:
• NAYAK, Pranati
Thuwal 23955-6900 (SA)
• ALSHAREEF, Husam Niman
Thuwal 23955-6900 (SA)
• BAEUMNER, Antje J.
93096 Koefering (DE)
• DUERKOP, Axel
84088 Neufahrn in Niederbayern (DE)
• FENZL, Christoph
93047 Thyrnau (DE)
• HIRSCH, Thomas
93047 Regensburg (DE)

(74) Representative: Ipsilon
Le Centralis
63, avenue du Général Leclerc
92340 Bourg-la-Reine (FR)

(56) References cited:
JP-A- 2016 045 032

• LUO SIDA ET AL: "Direct laser writing for creating porous graphitic structures and their use for flexible and highly sensitive sensor and sensor arrays", CARBON, vol. 96, 28 September 2015 (2015-09-28), pages 522-531, XP029314389, ISSN: 0008-6223, DOI: 10.1016/J.CARBON.2015.09.076
• KATIE GRIFFITHS ET AL: "Laser-scribed graphene presents an opportunity to print a new generation of disposable electrochemical sensors", NANOSCALE, vol. 6, no. 22, 24 September 2014 (2014-09-24), pages 13613-13622, XP055403122, United Kingdom ISSN: 2040-3364, DOI: 10.1039/C4NR04221B
• VERONICA STRONG ET AL: "Patterning and Electronic Tuning of Laser Scribed Graphene for Flexible All-Carbon Devices", ACS NANO, vol. 6, no. 2, 28 February 2012 (2012-02-28), pages 1395-1403, XP055197734, ISSN: 1936-0851, DOI: 10.1021/nn204200w

- **LAI TING ET AL:** "Easy processing laser reduced graphene: A green and fast sensing platform for hydroquinone and catechol simultaneous determination", ELECTROCHIMICA ACTA, vol. 138, 18 June 2014 (2014-06-18), pages 48-55, XP029043470, ISSN: 0013-4686, DOI: 10.1016/J.ELECTACTA.2014.06.070
- **JEE Y. HWANG ET AL:** "Direct preparation and processing of graphene/RuO 2 nanocomposite electrodes for high-performance capacitive energy storage", NANO ENERGY, vol. 18, 25 September 2015 (2015-09-25), pages 57-70, XP055403129, ISSN: 2211-2855, DOI: 10.1016/j.nanoen.2015.09.009
- **CHRISTOPH FENZL ET AL:** "Laser-Scribed Graphene Electrodes for Aptamer-Based Biosensing", ACS SENSORS, vol. 2, no. 5, 25 April 2017 (2017-04-25), pages 616-620, XP055403097, ISSN: 2379-3694, DOI: 10.1021/acssensors.7b00066
- **PRANATI NAYAK ET AL:** "Highly Efficient Laser Scribed Graphene Electrodes for On-Chip Electrochemical Sensing Applications", ADVANCED ELECTRONIC MATERIALS, vol. 2, no. 10, 11 August 2016 (2016-08-11), page 1600185, XP055403099, ISSN: 2199-160X, DOI: 10.1002/aelm.201600185

## Description

### CROSS-REFERENCE TO RELATED APPLICATIONS

This application claims the benefit of and priority to U.S. Provisional Application Serial No. 62/364,917, having the title "ON-CHIP GRAPHENE ELECTRODE, METHODS OF MAKING, AND METHODS OF USE", filed on July 21, 2016.

### BACKGROUND

Numerous functional carbon materials have been investigated as electrodes for the detection of (bio)chemical analytes in electrochemical assays. The electroanalytical performance of these materials is strongly influenced by the structural properties of the carbon itself which is mainly attributed to the density of electronic states as well as the edge-plane sites available on the surface. Of all carbon materials, graphene has emerged as the most promising candidate.

The article "Direct laser writing for creating porous graphitic structures and their use for flexible and highly sensitive sensor and sensor arrays" by Luo Sida et al. (DOI: 10.1016/J.CARBON.2015.09.076) discloses a one-step direct laser writing method to produce flexible and conductive graphitic porous patterns or arrays from polyimide.

The article "Laser-scribed graphene presents an opportunity to print a new generation of disposable electrochemical sensors" by Katie Griffiths et al. (DOI: 10.1039/ C4NR04221B) discloses using laser-scribed graphene as a distinct electrode patterned on a non-conducting flexible substrate to achieve a fast heterogeneous electron transfer rate.

The article "Patterning and Electronic Tuning of Laser Scribed Graphene for Flexible All Carbon Devices" by Veronica Strong et al. (DOI: 10.1021/nn204200w) discloses an all-optical patterning of graphene based on two-photon oxidation that allows for introduction of a band gap and continuous tuning of electrical and optical properties.

JP 2016 045032 discloses a biomolecule detection element comprising an aptamer bound to a biomolecule to be detected and a fluorescent dye bound to one end of the aptamer.

### SUMMARY

The invention is defined by the appended claims. Embodiments of the present disclosure provide for on-chip electrode platform devices and methods of making on-chip electrode platform devices and the like, which can be used for biosensing of biological targets and the like.

An embodiment of the present disclosure includes a biosensing device that comprises an on-chip electrode platform disposed on a polymer substrate, comprising: a three-dimensional laser scribed graphene counter electrode; a three-dimensional laser scribed graphene working electrode; and a three-dimensional laser scribed graphene electrode, the electrodes being directly grown on the substrate, and the three-dimensional laser scribed graphene counter electrode, working electrode, and electrode having a self-standing macro-mesoporous three-dimensional morphology comprising a macroporous surface having a mesoporous network of pores. The three-dimensional laser scribed graphene working electrode includes Pt nanoparticles disposed on the three-dimensional laser scribed graphene surface. The three-dimensional laser scribed graphene working electrode includes 1-pyrenebutyric acid anchored to graphene of the three-dimensional laser scribed graphene working electrode and a bioreceptor covalently attached to a carboxyl group of the 1-pyrenebutyric acid.

An embodiment of the present disclosure also includes methods of making an on-chip electrode platform for biosensing. Electrodes of the on-chip electrode platform are formed on a polyimide substrate by directing a laser beam onto the polyimide substrate. The electrodes comprise a three-dimensional laser scribed graphene counter electrode, a three-dimensional laser scribed graphene working electrode, and a three-dimensional laser scribed graphene electrode. The three-dimensional laser scribed graphene counter electrode, working electrode, and electrode have a self-standing macro/mesoporous three dimensional morphology comprising a microporous surface having a mesoporous network of pores. Pt nanoparticles are disposed on the three-dimensional laser scribed graphene working electrode. 1-pyrenebutyric acid is anchored to the graphene of the three-dimensional laser scribed graphene working electrode. A bioreceptor is covalently attached to a carboxyl group of the 1-pyrenebutyric acid.

Other apparatus, structures, methods, features, and advantages will be or become apparent to one with skill in the art upon examination of the following drawings and detailed description.

### BRIEF DESCRIPTION OF THE DRAWINGS

Further aspects of the present disclosure will be more readily appreciated upon review of the detailed description of its various embodiments, described below, when taken in conjunction with the accompanying drawings.

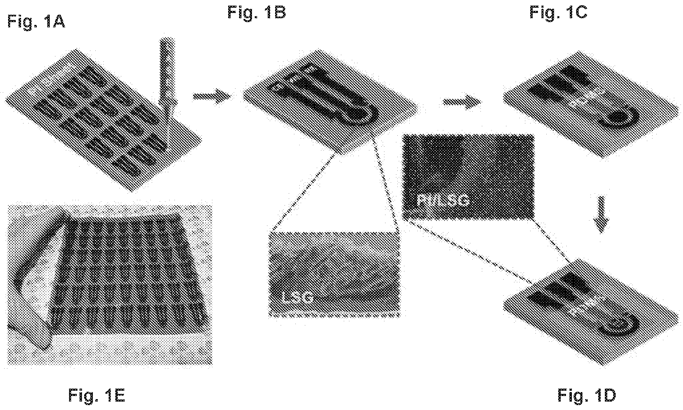

Figures 1A-E are a schematic illustration of the fabrication of LSG and Pt/LSG electrode pattern on PI sheet. Fig. 1A shows fabrication of arrays of electrodes on PI sheet by laser scribing and Fig. 1B is a 3D view of the LSG electrode pattern. The projection displays vertical cross-sectional SEM image of a laser-scribed PI sheet showing porous and protruded morphology of graphene, selective passivation of electrode area by a PDMS (Fig. 1 C). Fig. 1D shows deposition of Pt NPs selectively on working electrode area induced by electrochemical deposition technique. The projection displays SEM image of homogeneously anchored Pt NPs over graphene sheets. Fig 1E is a digital photograph of patterned electrode arrays on PI sheet.

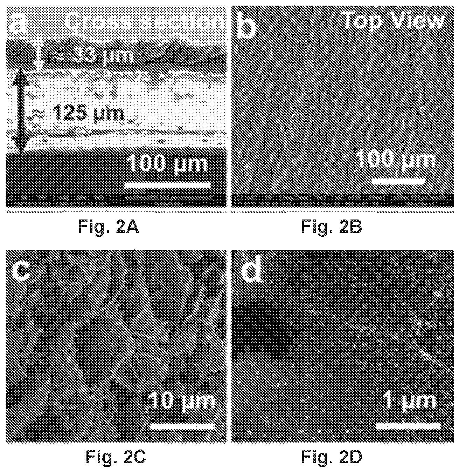

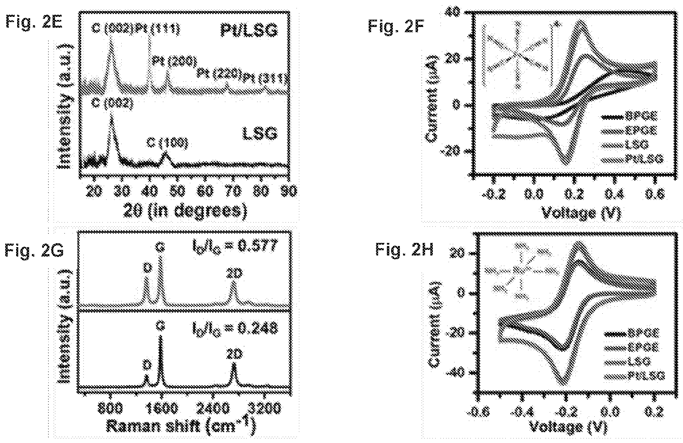

Figures 2A-D show the morphology of 3D porous graphitic carbon. Figures 2A-B show cross sectional and top-view SEM images of the LSG. Figures 2C-D are SEM images of Pt/LSG displaying anchored Pt NPs over LSG at different magnifications. Figure 2E shows XRD patterns and Fig. 2G is Raman spectra of the LSG and Pt/LSG. Cyclic voltammograms analyses of LSG based electrodes. CVs for (Fig. 2F) 5 mM $[Fe(CN)_6]^{4-}$ and (Fig. 2H) 5 mM $[Ru(NH_3)_6]^{3+}$ on LSG and Pt/LSG compared with BPGE and EPGE at a scan rate 10 mV/s. All measurements were performed relative to external Ag/AgCl (3 M KCl) reference electrode and 0.1 M KCl solution was used as supporting electrolyte.

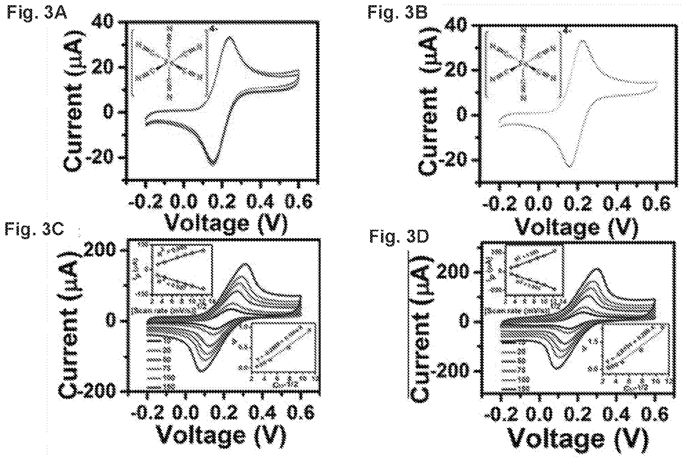

Figures 3A-D are cyclic voltammograms and allied kinetic analyses at LSG and Pt/LSG electrodes. Fig. 3A illustrates CVs for 5 mM $[Fe(CN)_6]^{4-}$ at 10 mV/s scan rate for 5 different LSG electrodes. Fig. 3D illustrates repeat CVs for 5 mM $[Fe(CN)_6]^{4-}$ at 10 mV/s scan rate on LSG electrode. Each cycle was run at 5 min intervals; a total of 20 cycles. CVs of (Fig. 3C-D) 5 mM $[Fe(CN)_6]^{4-}$ at different scan rates for LSG and Pt/LSG electrodes in 0.1 M KCl solution as supporting electrolyte. Insets (upper left) show plot of measured redox peak current ($I_\rho$) vs. square root of scan rate ($v^{1/2}$). Inset (lower right): plot of Nicholson's kinetic parameter $\psi$ versus C multiplied by reciprocal of the square root of the scan rate ($Cv^{-1/2}$). Linear fitting is used to calculate the standard heterogeneous charge transfer rate constant ($k_0$). All measurements were performed relative to external Ag/AgCl (3 M KCl) reference electrode.

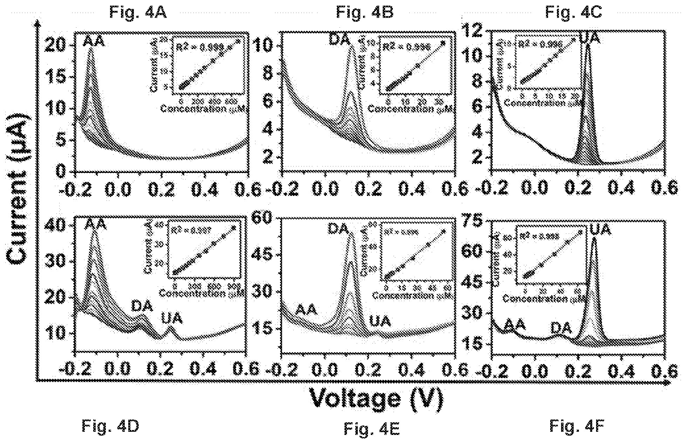

Figures 4A-F show electrochemical detection of AA, DA, and UA. Differential pulse voltammetry (DPV) at LSG at different concentrations of (Fig. 4A) AA, (Fig. 4B) DA and (Fig. 4C) UA in 0.1 M PBS (pH 7.0) as supporting electrolyte. DPV at Pt/LSG for various concentrations of (Fig. 4D) AA in a mixture of 4 $\mu$M DA and 4 $\mu$M UA, (Fig. 4E) DA in a mixture of 30 $\mu$M AA and 4 $\mu$M UA, and (Fig. 4F) UA in a mixture of 40 $\mu$M AA and 4 $\mu$M DA. Insets: plots of the oxidation peak current vs concentration of each biomolecule. Linear fitting is used to determine the sensitivity of the electrodes for each biomolecule. DPV conditions: pulse height = 50 mV, pulse width = 0.2 s, step height = 4 mV, step time = 0.5 s and scan rate = 8 mV/s. All measurements were performed relative to external Ag/AgCl (3 M KCl) reference electrode.

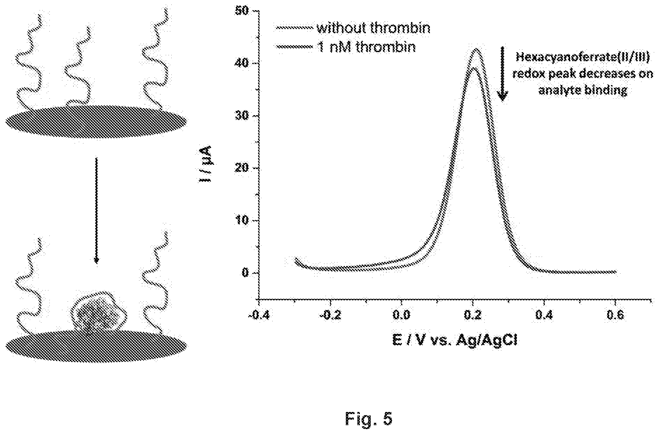

Figure 5 shows sensing scheme for aptamer-based thrombin detection. Upon binding of thrombin to the anti-thrombin aptamer-modified LSG electrode (left), the diffusion of the redox marker (here hexacyanoferrate(II/III) was used) to the electrode is hindered. This leads to a decrease in the peak current of differential pulse voltammetry (DPV) measurements (right).

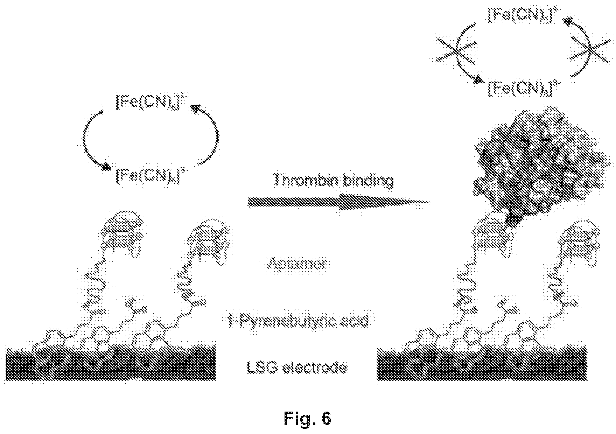

Figure 6 is a schematic of the electrochemical thrombin detection mechanism. With increasing concentrations of thrombin, the diffusion of the redox marker hexacyanoferrate(III) to the electrode surface is increasingly hindered. This effect decreases the peak currents obtained from voltammetric measurements.

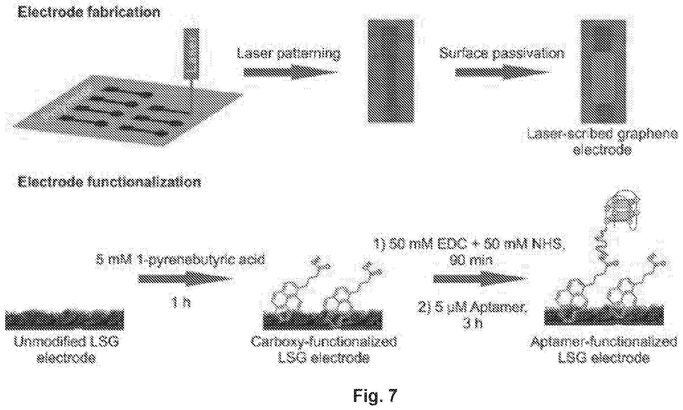

Figure 7 shows the electrode fabrication and functionalization process.

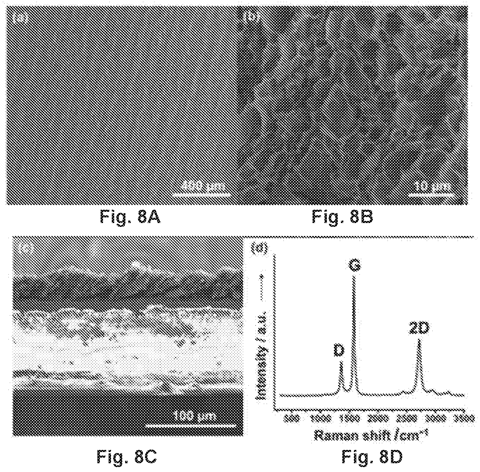

Figures 8A-D provide characterization of the LSG electrodes. Figures 8A-B are SEM images of the top view of the LSG electrode. Figure 8C is a cross-sectional SEM image of the same electrode. Figure 8D shows the Raman spectrum of laser-scribed graphene.

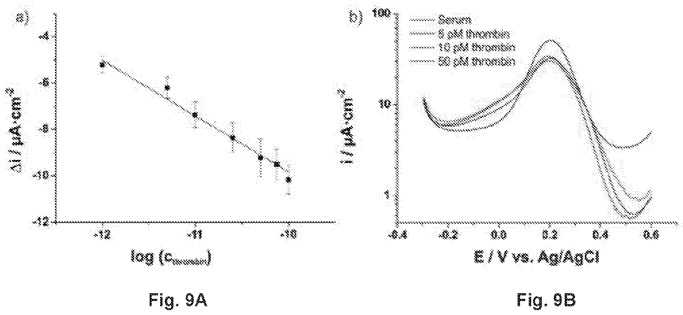

Figure 9A shows the change of the DPV peak current of PBA-modified LSG electrode after aptamer immobilization vs. Ag/AgCl at varying thrombin concentration in phosphate buffered saline at pH 7.4 containing 5 mM $K_3[Fe(CN)_6]$. n=3. Figure 9B shows differential pulse voltammograms in phosphate buffered saline at pH 7.4 containing 5 mM $K_3[Fe(CN)_6]$ of PBA-modified LSG electrode after aptamer immobilization vs. Ag/AgCl at varying thrombin concentration in serum.

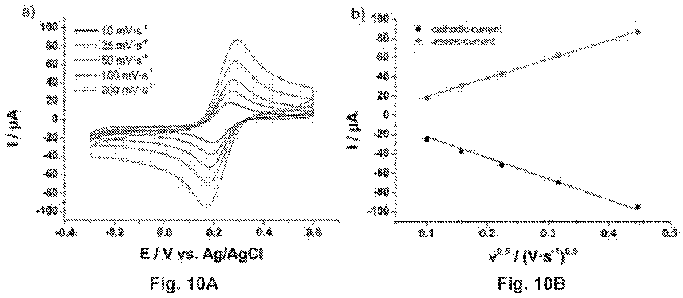

Figure 10A shows cyclic voltammograms of LSG electrode vs. Ag/AgCl in PBS (at pH 7.4 containing 5 mM $K_3[Fe(CN)_6]$) at varying scan rates from 10 to 200 mV·s$^{-1}$. Figure 10B is a Randles-Sevcik plot of LSG electrode vs. Ag/AgCl in PBS at pH 7.4 containing 5 mM $K_3[Fe(CN)_6]$ at varying scan rates from 10 to 200 mV·s$^{-1}$.

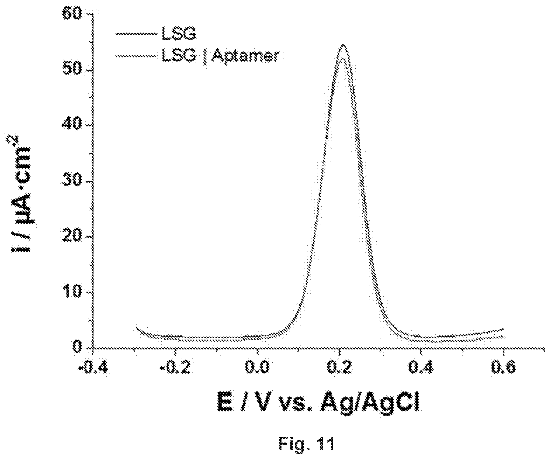

Figure 11 provides differential pulse voltammograms of a LSG electrode without PBA modification in PBS at pH 7.4 containing 5 mM $K_3[Fe(CN)_6]$ before and after attachment of anti-thrombin aptamer. The peak current density drops only 4.7$\pm$0.4% due to insufficient functionalization with the aptamer.

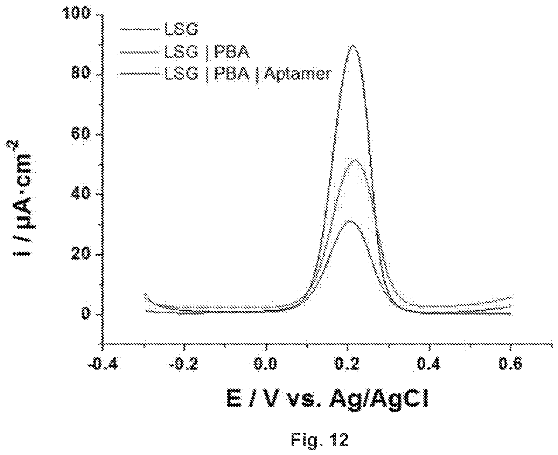

Figure 12 provides differential pulse voltammograms of a LSG electrode in PBS at pH 7.4 containing 5 mM $K_3[Fe(CN)_6]$ before and after modification with 1-pyrenebutyric acid (PBA), and after further modification with anti-thrombin aptamer. The addition of PBA and aptamer to the electrode leads to a decrease in the peak current density due to hindered diffusion of the redox marker.

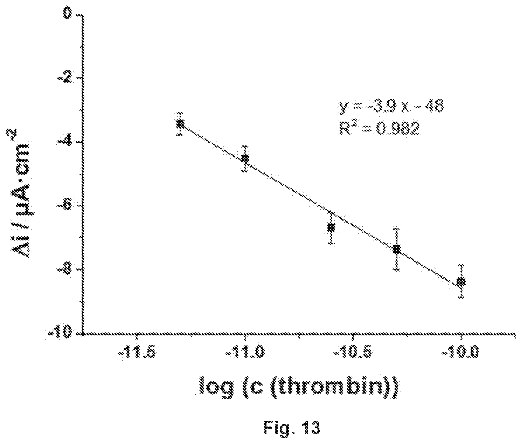

Figure 13 shows the change of the DPV peak current in phosphate buffered saline at pH 7.4 containing 5 mM $K_3[Fe(CN)_6]$ of 1-pyrenebutyric acid-modified LSG electrode after aptamer immobilization vs. Ag/AgCl at varying thrombin concentration in PBS containing 40 g·L$^{-1}$ bovine serum albumin. n=3.

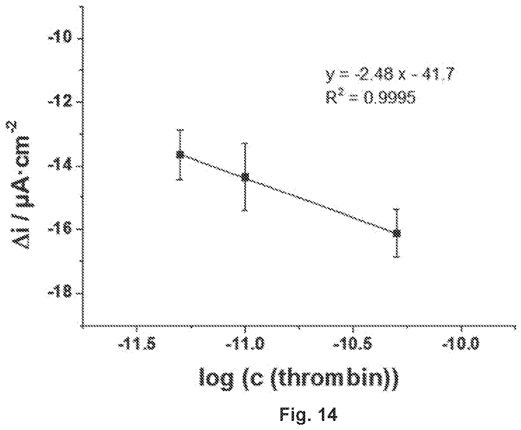

Figure 14 shows the change of the DPV peak current in PBS at pH 7.4 containing 5 mM K$_3$[Fe(CN)$_6$] of 1-pyrenebutyric acid-modified LSG electrode after aptamer immobilization vs. Ag/AgCl at varying thrombin concentration in serum. n=3.

## DETAILED DESCRIPTION

This disclosure is not limited to particular embodiments described, and as such may, of course, vary. The terminology used herein serves the purpose of describing particular embodiments only, and is not intended to be limiting, since the scope of the present invention is limited only by the appended claims.

Where a range of values is provided, each intervening value, to the tenth of the unit of the lower limit unless the context clearly dictates otherwise, between the upper and lower limit of that, range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Embodiments of the present disclosure will employ, unless otherwise indicated, techniques of material science, chemistry, biology, and the like, which are within the skill of the art. Such techniques are explained fully in the literature.

The following examples are put forth so as to provide those of ordinary skill in the art with a complete disclosure and description of how to perform, make, and use the embodiments disclosed and claimed herein. Efforts have been made to ensure accuracy with respect to numbers (*e.g.*, amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, dimensions, frequency ranges, applications, or the like, as such can vary. It is also to be understood that the terminology used herein is for purposes of describing particular embodiments only, and is not intended to be limiting. It is also possible in the present disclosure that steps can be executed in different sequence, where this is logically possible. It is also possible that the embodiments of the present disclosure can be applied to additional embodiments involving measurements beyond the examples described herein, which are not intended to be limiting. It is furthermore possible that the embodiments of the present disclosure can be combined or integrated with other measurement techniques beyond the examples described herein, which are not intended to be limiting.

It should be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

Definitions:

"Aptamers" may be high affinity, high specificity polypeptide, RNA, or DNA-based probes produced by in vitro selection experiments. Aptamers may be generated from random sequences of nucleotides or amino acids, selectively screened by absorption to molecular antigens or cells, and enriched to purify specific high affinity binding ligands, for example. In solution, aptamers may be unstructured but may fold and enwrap target epitopes providing specific binding recognition. The unique folding of the nucleic acids around the epitope, for example, affords discriminatory intermolecular contacts through hydrogen bonding, electrostatic interaction, stacking, and shape complementarity.

Use of the phrase "biomaterial" or "biomolecule" is intended to encompass at least deoxyribonucleic acid (DNA), ribonucleic acid (RNA), nucleotides, oligonucleotides, nucleosides, polynucleotides, proteins, peptides, polypeptides, selenoproteins, antibodies, antigens, protein complexes, aptamers, combinations thereof, and the like.

Use of "biological target" is intended to encompass biomolecules (e.g., deoxyribonucleic acid (DNA), ribonucleic acid (RNA), nucleotides, oligonucleotides, nucleosides, polynucleotides, proteins, peptides, polypeptides, selenoproteins, antibodies, antigens, protein complexes, aptamers, combinations thereof), and the like. In particular, biological target can include, but is not limited to, naturally occurring substances such as polypeptides, polynucleotides, lipids, fatty acids, glycoproteins, carbohydrates, fatty acids, enzymes, fatty esters, macromolecular polypeptide complexes, vitamins, co-factors, whole cells, eukaryotic cells, prokaryotic cells, micelles, microorganisms such as viruses, bacteria, protozoa, archaea, fungi, algae, spores, apicomplexan, trematodes, nematodes, mycoplasma, or combinations thereof. In addition, the biological target can include native intact cells, viruses, bacterium, and the like.

Use of the term "affinity" can include biological interactions and/or chemical interactions. The biological interactions can include, but are not limited to, bonding or hybridization among one or more biological functional groups located on the first biomolecule or biological target and the second biomolecule or biological target. The chemical interaction can include, but is not limited to, bonding among one or more functional groups (*e.g.*, organic and/or inorganic functional groups) located on the biomolecules.

As used herein, "targeting agent" is intended to encompass any molecule, including but not limited to aptamers, a chemical agent, a biological agent (*e.g.*, polypeptides (*e.g.*, proteins such as, but not limited to, antibodies (monoclonal or polyclonal), fragments of antibodies), antigens, nucleic acids (both monomeric and oligomeric), peptoids, polysaccharides, haptens, sugars, fatty acids, steroids, purines, pyrimidines, ligands, and aptamers) and combinations thereof, having an affinity for a biological target.

Discussion:

Embodiments of the present disclosure provide a device including an on-chip electrode platform including one or more three dimensional laser scribed graphene electrodes, methods of making the on-chip electrode platform, methods of analyzing (*e.g.*, detecting, quantifying, and the like) chemicals and biochemicals, and the like. Embodiments of the present disclosure relate to large scale fabrication of an on-chip electrode platform fabricated by direct growth of a porous binder free three dimensional graphene architectures on substrates (*e.g.*, polyimide) employing laser scribing of the surface of the substrate.

In particular, embodiments of the present disclosure include large scale flexible electrochemical sensors that can be fabricated by adopting direct growth of graphitic carbon patterns on a substrate, such as a commercial polyimide surface, by use of a laser scribing approach, where the material modification can be referred to as laser scribed graphene (LSG). Embodiments of the present disclosure are beneficial in that large-scale fabrication of a chemical or biosensor device with three dimensional porous binder free graphene networks as active material can be produced, where the on-chip electrodes of the platform exhibit superior electrochemical activity and sensing properties. In an embodiment, the on-chip electrodes can be used to precisely monitor the dopamine, ascorbic acid and/or uric acid levels in a sample(s) with minimum interference, which is described in more detail in the Examples. As compared to some systems, embodiments of the present disclosure are free from enzymes, so that the on-chip electrode platform can be stored at room temperature and reused several times without any passivation effect.

An embodiment of the device includes an on-chip electrode platform that is disposed on a substrate. The on-chip electrode platform includes a plurality of electrodes, where at least one of the electrodes is a three dimensional laser scribed graphene electrode that has a self-standing macro/mesoporous three dimensional morphology. The laser scribed graphene includes self-standing macro/mesoporous three dimensional morphology with ample amount (*e.g.*, greater than about 5%, greater than about 10%, greater than about 15%, greater than about 20%, greater than about 25%, about 5 to 30%, about 15 to 30%, about 15 to 25%, or about 19 to 22%) of edge plane sites and large (*e.g.*, the surface area determined by the Randles-Sevcik plot (Figure 10B) is $1.3 \pm 0.2$ cm$^2$, which is more than 18-fold the physical area (0.07 cm$^2$) and is due to the lamella-like structure of the LSG (Figures 8A-C)) electrochemical active surface area, facilitates ion diffusion leading to efficient electron transfer events.

In an embodiment, the macro/mesoporous three dimensional morphology includes a macroporous surface with a mesoporous porous architecture superimposed on the surface of the macroporous surface. In other words, there is a mesoporous network of pores on the macroporous surface. In an embodiment, the macroporous surface includes pores having a diameter of about 50 nm or more (*e.g.*, in the $\mu$m range or higher) and the mesoporous porous architecture includes pores having a diameter of about 2 nm to 10 nm. In an embodiment, the macropores can extend to a depth of about 10 $\mu$m to 50 $\mu$m of the electrode. In an embodiment, the pores can be straight or winding and/or branched or unbranched and the diameter can vary at different depths of the pores.

In an embodiment, the self-standing macro/mesoporous three dimensional morphology can include a three dimensional network of micron size graphene flakes. The macro porosity of the network facilitates electrolyte diffusion into porous network that results in about a 30% enhancement of electrochemical active surface area compared to physical surface area of the LSG electrode. The cross-sectional SEM images reveal an ordered-porous morphology, and including this, the graphene flakes exhibit mesoporous structures that facilitates ion diffusion leading to better catalytic activity compared to commercial graphite electrodes.

In an embodiment, the substrate can be a material that forms three dimensional laser scribed graphene upon exposure to a laser beam. The focused laser beam produces very high local temperatures (*e.g.*, greater than about 2500 °C), which helps in carbonization and graphitization of the surface of the substrate. In an embodiment, the substrate can be a substrate having polyimide polymer surface thick enough to form the electrode. In an embodiment, the polymer layer or the polymer substrate can include a polymer having heterocyclic structures such as the imide group. In an embodiment, the polymer layer or the polymer substrate can include: polyimide, as well as other polymers including heterocyclic structures (*e.g.*, imide group) or a combination thereof.

In an embodiment, the three dimensional laser scribed graphene electrode can have a thickness of about 10 $\mu$m to 50 $\mu$m or about 30 to 36 $\mu$m or about 33 $\mu$m. The other dimensions can be appropriate for the electrode being formed as well as the overall design of the electrode platform, so that so length and width can be selected according to the design of the electrode platform, for example the width can vary as a function of location on the electrode. In general the length and width are on the order of mm's to cm's. Figures 1(a) to 1(e) illustrate that the dimensions can be selected according to the design of the electrode platform.

In an embodiment, the device includes a multi-electrode platform (*e.g.*, a three electrode platform (counter electrode (CE), working electrode (WE) and electrode (E or RE)) where each electrode is made up of three dimensional porous graphene sheets patterned over substrate (*e.g.*, See Figures 1A-E). Each of the electrodes can have the same or different dimensions.

In an embodiment, one or more of the electrodes can include one or more types of particles disposed on a surface of the electrode to modify the functioning of the electrode. For example, the particles may enhance sensitivity and/or selectivity to one or more chemical or biochemical species of interest. In an embodiment, an area of the working electrode can be defined (isolated) by selective passivation from rest of the pattern (*e.g.*, by PDMS coating) so that, the working electrode can be selectively modified on a selected surface area, or the reactive portion of the electrode.

For example, the working electrode can be modified by selective disposition (*e.g.*, anchoring (*e.g.*, attaching or bonding to the nanoparticles over the graphene layer)) of metal nanoparticles (*e.g.*, Pt nanoparticles) by electrodeposition or other appropriate technique that accomplishes the same or similar result. In an embodiment, the particles can include metal nanoparticles such as transition metal nanoparticles, Pt nanoparticles, Cu nanoparticles, Ni nanoparticles, Zn nanoparticles, Ag nanoparticles, Au nanoparticles, Pd nanoparticles, Ru nanoparticles, oxides of each (*e.g.*, CuO, NiO, ZnO), metal hydroxides of each (*e.g.*, $Cu(OH)_2$, $Ni(OH)_2$), oxynitrides of each, and the like. In an embodiment, the nanoparticles have a diameter (for spherical-shaped particles, and if non-spherical, then these dimension correspond to each of height, length, and width) of about 1 to 500 nm, about 10 to 250 nm, or about 10 to 100 nm.

In another embodiment, the working electrode can be modified by selective disposition (*e.g.*, anchoring (*e.g.*, attaching or bonding to the graphene layer)) of a targeting agent to the surface of the working electrode. In an embodiment, the targeting agent can attach (*e.g.*, be bound to the substrate) to (directly or indirectly) the working electrode. In an embodiment, the targeting agent can include biomolecules (e.g., deoxyribonucleic acid (DNA), ribonucleic acid (RNA), nucleotides, oligonucleotides, nucleosides, polynucleotides, proteins, peptides, polypeptides, selenoproteins, antibodies, antigens, protein complexes, aptamers, combinations thereof), and the like. In particular, the biological target can include, but is not limited to, naturally occurring substances such as polypeptides, polynucleotides, lipids, fatty acids, glycoproteins, carbohydrates, fatty acids, enzymes, fatty esters, macromolecular polypeptide complexes, vitamins, co-factors, whole cells, eukaryotic cells, prokaryotic cells, micelles, microorganisms such as viruses, bacteria, phage, protozoa, archaea, fungi, algae, spores, apicomplexan, trematodes, nematodes, mycoplasma, or combinations thereof. In addition, the biological target can include native intact cells, viruses, bacterium, and the like. In general, the targeting agent has an affinity for a biological target that is to be detected.

In an embodiment, the targeting agent can be attached or bonded (directly or indirectly) to the surface of the working electrode by a physical, biological, biochemical, and/or chemical association. The terms "attach" or "bond" can include, but are not limited to, chemically bonded (*e.g.*, covalently or ionically), biologically bonded, biochemically bonded, and/or otherwise associated with the particle. In an embodiment, bond can include, but is not limited to, a covalent bond, a non-covalent bond, an ionic bond, a chelated bond, as well as being bound through interactions such as, but not limited to, hydrophobic interactions, hydrophilic interactions, charge-charge interactions, $\pi$-stacking interactions, combinations thereof, and like interactions.

In an embodiment, the targeting agent can be directly bonded to the surface of the working electrode and/or to particles on the surface of the working electrode.

In an embodiment, the targeting agent can be indirectly bonded to the surface of the working electrode and/or to particles using a linking agent. The linking agent functions to bond to the working electrode and/or to particle and also bond to the targeting agent. The linking agent includes, but is not limited to, particles, nanomaterials, and molecules binding covalently (such as diazonium salts, nitrophenyls and benzoyl peroxide) or non-covalently to the graphene surface. In the latter case, molecules can bind to the graphene via $\pi$-$\pi$ stacking, ionic bonding, hydrogen bonding, hydrophobic or electrostatic interactions. Examples include polymer wrapping, adsorption of surfactants, and binding of small aromatic compounds. Examples include sulfonated polyaniline, poly(sodium 4-styrenesulfonate), pyrene butyric acid.

Embodiments of the present disclosure can be fabricated by adopting direct growth of graphitic carbon patterns on a substrate employing a laser scribing approach. The focused laser beam could produce very high local temperatures (*e.g.*, greater than about 2500 °C), which helps in carbonization and graphitization of the surface of the substrate (*e.g.*, polymer surface) to form the electrode platform. The laser(s) can be used to form one or more three dimensional laser scribed graphene electrode that include self-standing macro/mesoporous three dimensional morphology. The time frame of exposure as well as the power of the laser can be used to form the desired dimensions and/or characteristics of the electrode(s). In an embodiment, the laser can include commercially used lasers that are in the 2.4 to 5.4 W range. In an embodiment, the time of exposure of the surface to the laser can be about 0.003 to 1.3 s.

As mentioned above, particles, such as nanoparticies, can be disposed on one or more areas of the three dimensional laser scribed graphene electrode. In an embodiment, one or more types of particles (*e.g.*, metal nanoparticles) can be disposed on one or more areas of the three dimensional laser scribed graphene electrode. In an embodiment, Pt nanoparticles can be disposed on a working electrode. In an embodiment, the particles can be disposed using electrodeposition, electrodeposition, chemical reduction, microwave irradiation, or hydrothermal to anchor the particles to the electrode surface.

As mentioned above, embodiments of the present disclosure can be used to detect one or more chemical or biochemical. Embodiments of the present disclosure exhibit superior electrochemical activity and sensing properties as compared to some techniques. As describe in detail in the Example, an embodiment of the on-chip platform including three electrodes can be used to monitor the dopamine, ascorbic acid and/or uric acid levels in a sample(s) with minimum interference, where the results are comparable or superior to other techniques. Also, due to the simplicity of the present disclosure, the on-chip electrode platform can be stored at room temperature and reused.

In electrochemical sensing applications and commercialization, the electrodes having high electrochemically active surface area, good charge transfer behavior, a broad detection range with high sensitivity and selectivity and finally a scaled up fabrication technique is highly desired.

In an embodiment, the average charge transfer coefficient ($k^0$) value for laser scribed graphene electrode can be about $0.0044 \, cm \cdot s^{-1}$ (*e.g.*, $0.0044 \pm 0.0003 \, cm \cdot s^{-1}$) for hexacyanoferrate (II/III) in PBS, demonstrating a favorable electron transfer rate in the case of three dimensional porous laser scribed graphene electrode. The enhancement is believed to be a combined consequence of the porous, binder free three dimensional network of laser scribed graphene electrode with large intrinsic protrusions of graphene sheets and rich in edge plane sites which, in turn, activate the graphene surface toward enhanced electrochemical reactions. In an embodiment, the charge transfer coefficient was further enhanced by incorporation of Pt NPs on laser scribed graphene electrode. The calculated $k^0$ value for Pt/ laser scribed graphene electrode is 0.2823 cm/s which elucidates the catalytic behavior of Pt NPs on the surfaces of the laser scribed graphene electrode facilitating the charge transfer mechanism.

In an embodiment, the laser scribed graphene electrode based electrode patterns have exhibited significantly improved electrocatalytic performance toward oxidation of ascorbic acid, dopamine and uric acid displaying high sensitivity, selectivity in a wide concentration range (See Figures 4A-F and Table 2). In an embodiment, laser scribed graphene electrode has been used for the detection of thrombin, which indicate that the laser scribed graphene electrode can be used for nM level thrombin detection.

Embodiments including high electrochemically active surface area, efficient charge transfer behavior and high repeatability of electrode material, provide for laser scribed graphene electrodes that can be used as a potential electrode material for different bio-analyte detection in broad range. Also, the laser based scaled-up fabrication technique provides the advantage in large scale commercialization for practical application.

## Examples

*Example 1*

The following examples are directed to large scale fabrication of on-chip electrode platform based on direct growth of porous binder free three dimensional graphene architectures on commercial polyimide (PI) sheet employing laser scribing and its application towards detection of biomolecules. An embodiment of the on-chip electrode platform is provided in Figures 1A-E.

Figure 1A illustrates that large scale flexible electrochemical sensors can be fabricated by adopting direct growth of graphitic carbon patterns on a commercial polyimide surface employing a laser scribing approach (LSG: laser scribed graphene). Characteristics of LSG include self-standing macro/mesoporous 3D morphology with ample amount of edge plane sites and large electrochemical active surface area, facilitates ion diffusion leading to efficient electron transfer events.

The on-chip device structure includes a three-electrode platform (counter electrode (CE), working electrode (WE) and electrode (E or RE)) made up of 3D porous graphene sheets patterned over flexible PI sheet, as shown in Figure 1B and 2A and 2B. The working electrode area has been defined by selective passivation from rest of the pattern by PDMS coating as shown in Figure 1C. Further the functional behavior of the LSG WE has been modified by selective anchoring of Pt nanoparticles by electrodeposition (Figure 1D and 2C and 2D). The focused laser beam could produce very high local temperatures (> 2500 °C), which helps in carbonization and graphitization of polymer surfaces. Further electrodeposition leads successful anchoring of Pt NPs over LSG, which were confirmed by different characterization techniques such as X-ray diffraction and Raman spectra analyses (Figure 2E and 2G).

The electrochemistry of LSG based electrodes were thoroughly investigated before implementing it as a biosensor platform. Figures 2F and 2H show CV response for LSG and Pt/LSG electrodes for two different redox probes $[Fe(CN)_6]^{4-}$ and 5 mM $[Ru(NH_3)_6]^{3+}$ and compared with commercial basal plane graphite electrode (BPGE) and edge plane graphite electrode (EPGE), respectively. A lesser $\Delta E_p$ value with a large redox current ($I_p$) compared to EPPG and BPGE, indicates faster charge transfer mechanism in LSG and Pt/LSG based electrodes. (Figures 2F and 2H)

The electrochemical active surface area (A) for LSG and Pt/LSG electrodes were estimated based on the Randles-Sevcik equation, which assumes mass transport only by diffusion process.

$$Ip = 2.69 \times 10^5 \, AD^{1/2}n^{3/2}\upsilon^{1/2}C \quad (1)$$

Where n is the number of electrons participating in the redox reaction, D is the diffusion coefficient of the molecule (D = $6.70 \times 10^{-6}$ cm$^2$/s for [Fe(CN)$_6$]$^{4-}$ and $8.43 \times 10^{-6}$ cm$^2$/s for [Ru(NH$_3$)$_6$]$^{3+}$), C is the concentration of the probe molecule in the solution (mol/cm$^3$), and is the scan rate (V/s). Using the experimental parameters, the calculated electrochemical active surface area A value for the LSG electrode was found to be ~9.177 mm$^2$ which is ~ 30% enhancement compared to physical surface area of the LSG electrode (7.065 mm$^2$). Also the calculated average A value for the Pt/LSG is ~10.383 mm$^2$. Thus, the addition of Pt nanoparticles increased the effective electrode surface area by roughly 13.14%.

A reproducible redox current observed for five different LSG electrodes indicates the repeatability of the fabricated microelectrodes (Figure 3A). No significant change in $\Delta E_p$ value or deterioration in $I_p$ was observed in CVs recorded at 5 minute intervals for up to 20 cycles (Figure 3B), indicates no surface passivation/blocking of the electrode surface by repeated cycling of electrode material.

The average charge transfer coefficient ($k_0$) value is calculated to be 0.1150 cm/s (Figure 3C) for [Fe(CN)$_6$]$^{4-}$ in LSG electrode, demonstrating a favorable electron transfer rate in the case of 3D porous LSG. Being the edge plane electron transfer kinetics are anomalously faster than that of inert basal plane; the enhancement is believed to be a combined consequence of the porous, binder free 3D network of LSG with large intrinsic protrusions of graphene sheets and rich in edge plane sites which, in turn, activate the graphene surface toward enhanced electrochemical reactions. Further, the calculated $k_0$ value for Pt/LSG (Figure 3D) is 0.2823 cm/s which elucidates the catalytic behavior of Pt NPs on the surfaces of the LSG facilitating the charge transfer mechanism. The calculated $K^0$ values are promising compared to similar carbon based electrode materials in literature as shown in Table 1.

Table 1. Comparison of charge transfer kinetics of LSG and Pt/LSG based electrodes with similar carbon based electrodes in literature.

| Electrodes | $k^0$ (cm/s) | | References |
|---|---|---|---|
| | $[Fe(CN)_6]^{4-}$ | $[Ru(NH_3)_6]^{3+}$ | |
| Pt/LSG | 0.2823 | 0.2312 | Present work |
| LSG | 0.1150 | 0.0868 | Present work |
| BPPG | $10^{-9}$ | 0.0038 | [1], [2] |
| EPPG | 0.022 | 0.00877 | [1], [2] |
| L-graphene | 0.02373 | - | [3] |
| CVD-Gr | 0.014 | 0.012 | [4] |
| Q-graphene | 0.0186 | 0.0177 | [5] |
| q-graphene | - | 0.00158 | [2] |
| m-graphene | - | 0.0011 | [2] |

Pt/LSG: Pt nanoparticles decorated laser scribed graphene

LSG: Laser scribed graphene

BPPG: Basal plane graphite electrode

EPPG: Edge plane graphite electrode

L-graphene: Laser induced graphene from GO

CVD-Gr: graphene grown on copper foil by chemical vapor deposition

Q-graphene: polyhedral structure graphene

q-graphene: few-layer (termed quasi-) graphene grown via CVD

m-graphene: mono-layer graphene grown via CVD

As described herein, embodiments of the present disclosure pertain to the large-scale fabrication of on-chip electrode platforms based on direct growth of porous binder free three dimensional graphene architectures on commercial PI sheet its application towards biosensing. Based on the above discussed electrochemical performances, the LSG based electrode was employed for the potential detection of biologically promising analytes such as dopamine (DA), ascorbic acid (AA) and/or uric acid (UA). DA is one of the important neurotransmitter in the mammalian central nervous system. Any abnormality leads to severe neurological disorders such as Parkinson's disease and Schizophrenia, and hence is a key marker for the treatment of these diseases. AA is well-known for its antioxidant properties and plays a key role in biological metabolism. UA is an important natural antioxidant produced when the body breaks down purines. Abnormal levels of UA are symptomatic of hyperuricemia and several kidney diseases. Because UA, DA, and AA coexist in the central nervous system and the electrochemical oxidation peak potentials overlap at conventional solid electrodes, the primary challenge encountered here is to separate their oxidation peak potentials with enhanced intensity.

The typical DPV response of the LSG electrode to the addition of different concentrations of DA, UA and AA in PBS solution is shown in Figures 4A-C. The oxidation current increased linearly as the concentration is increased from 10-680 $\mu$M for AA, 0.5-32.5 $\mu$M for DA and 0.5-19.5 $\mu$M for UA, respectively. Since UA, DA, and AA coexist in biological fluid, the accurate measurements of each component with a high degree of selectivity are highly enviable. We studied the performance of Pt/LSG electrode at various concentrations for each species with the other two species held at fixed concentrations. Figure 4D-F represents the DPV after repeatedly adding different concentrations of AA, UA and DA while keeping the other two fixed. The current is seen increasing linearly as the concentration is increased from 10-890 $\mu$M for AA, 0.5-56 $\mu$M for DA and 1-63 $\mu$M for UA for Pt/LSG electrode. It is noticed that the oxidation peak currents remain almost constant for two of the molecules at the expense of selective increase in the current when the concentration of the third one was increased, indicating no significant influence or interference of one species on other two. Very similar trends were observed for the detection of DA and UA, which is a signature of anti-interference ability (selectivity) of LSG based electrode. The LODs for Pt/LSG were 6.1, 0.07, and 0.22 $\mu$M for AA, DA and UA respectively. The sensitivity parameters were calculated from the slope of calibration plot to be 250 $\mu$A/mMcm$^2$, 7000 $\mu$A/mMcm$^2$ and 8300 $\mu$A/mMcm$^2$ for AA, DA and UA respectively, which are better than those of other carbon nanomaterial-based electrodes in the literature. All of the analytical parameters obtained from Figures 4A-F are summarized and compared with the data from the literature (Table 2). Thus, LSG based electrode is a promising candidate for biosensing due to their selectivity and sensitivity.

Table 2 Summary of sensing parameters for detection of AA, DA and UA by DPV using different carbon based electrodes.

| Electrodes | Linearity range ($\mu$M) | | | Sensitivity ($\mu$A/mMcm$^2$) | | | LOD ($\mu$M) | | | References |
|---|---|---|---|---|---|---|---|---|---|---|
| | AA | DA | UA | AA | DA | UA | AA | DA | UA | |
| Pt/LSG | 10-890 | 0.5-56 | 1-63 | 250.69 | 6995.6 | 8289 | 6.1 | 0.07 | 0.22 | Present work |
| LSG | 10-680 | 0.5-32.5 | 0.5-19.5 | 237.76 | 2259.9 | 5405 | 7.3 | 0.27 | 0.38 | Present work |
| 3D G | - | Up to 25 | - | - | 619.6 | - | - | 0.025 | - | [6] |
| ZnO NWA/GF | 5-80 | 0.5-40 | 0.5-40 | 0.38 $\mu$A/$\mu$M | 4.81 $\mu$A/$\mu$M | 4.25 $\mu$A/$\mu$M | 5 | 0.5 | 0.5 | [7] |
| GONR | 0.1-8.5 | 0.15-12.2 | 0.15-11.4 | - | - | - | 0.06 | 0.08 | 0.07 | [8] |
| Pt/G | 0.15-34.4 | 0.03-8.13 | 0.05-11.85 | 0.3457 $\mu$A/$\mu$M | 0.9695 $\mu$A/$\mu$M | 0.4119 $\mu$A/$\mu$M | 0.15 | 0.03 | 0.05 | [9] |
| N-PCNPs | 80-2000 | 0.5-30 | 4-50 | - | - | - | 0.74 | 0.011 | 0.021 | [10] |
| CHI/VSG/PPy | - | 0.1-200 | - | - | 632.1 | - | - | 0.019 | - | [11] |
| 3D graphene/CNT | - | 2-64 | - | - | 470.7 | - | - | 0.02 | - | [12] |

The LSG electrodes were also used to detect thrombin successfully. In fact, our initial results show that it can detect thrombin down to the nano molar level (Fig. 5).

*Example 2*

Graphene as transducer material has produced some of the best-performing sensing approaches to date opening the door toward integrated miniaturized all-carbon point-of-care devices. Addressing this opportunity, laser-scribed graphene (LSG) electrodes are demonstrated here as highly sensitive and reliable biosensor transducers in blood serum analysis. These flexible electrodes with large electrochemical surface areas were fabricated using a direct-write laser process on polyimide foils. A universal immobilization approach is established by anchoring 1-pyrenebutyric acid to the graphene and subsequently covalently attaching an aptamer against the coagulation factor thrombin as an exemplary bioreceptor to the carboxyl groups. The resulting biosensor displays extremely low detection limits of 1 pM in buffer and 5 pM in the complex matrix of serum.

Numerous functional carbon materials have been investigated as electrodes for the detection of (bio)chemical analytes in electrochemical assays.[1-3] The electroanalytical performance of these materials is strongly influenced by the structural properties of the carbon itself which is mainly attributed to the density of electronic states as well as the edge-plane sites available on the surface.[4,5] Of all carbon materials, graphene has emerged as the most promising candidate.[6,7] Its water-dispersible derivatives, such as graphene oxide, are often used for the modification of electrodes but even when reduced through (physico)chemical means[8-10] the outstanding electrochemical properties of pristine graphene cannot be achieved due to numerous structural defects in graphene oxides.[11] To bridge the existing gap between lab research and commercial graphene-based biosensors, reliable, scalable fabrication and modification methods are still needed.[12] Here, standalone graphene electrodes can be a promising solution. El-Kady *et al.*[13] have introduced a novel and straightforward method to produce supercapacitors made of graphene by the direct laser reduction of graphene oxide with a standard LightScribe DVD optical drive. The resulting films - referred to as laser-scribed graphene (LSG) - show high electrical conductivity and specific surface area, are mechanically robust, and can be used directly as capacitor electrodes without the need of additives. In a following study,[14] Strong *et al.* built a low-cost graphene-based $NO_2$ gas sensor besting conventional carbon electrodes due to the LSG's superior electrical conductivity. Further, LSG can be easily printed, scaled, and is flexible.[15,16] Combined with the high electron transfer rates, small peak potential differences and a good peak current response,[5,13,14] it is extremely interesting for a broad range of biosensing applications including, but not limited to complex clinical diagnostics and implantable sensing arrays. LSG offers the possibility to create flexible, all-printed and implantable microarrays of electrodes with high surface area for the detection of multiple analytes such as biomarkers,[17] neurotransmitters,[18] proteins or other biomolecules.[19] To move LSG into the arena of biosensing, a reliable route for its modification with various biorecognition elements is required.[20] Here we demonstrate a universal modification route for LSG electrodes by anchoring 1-pyrenebutyric acid (PBA) via $\pi$-stacking and hydrophobic interactions to the LSG and subsequently covalently attaching bioreceptors to the carboxyl groups using standard coupling chemistry. An LSG-based electrochemical biosensor for thrombin (Figure 6) was developed to prove the viability of this strategy, as fast responding and miniaturized sensors for thrombin are urgently needed in point-of care diagnostics in order to replace existing and rather complex sensors and methods.

Laser-scribed graphene compares favorably to other carbon devices with respect to combining high electron transfer rates, small peak potential differences and a good peak current response with fast and scalable fabrication.[5,14] As it doesn't require fabrication masks or elaborate lithography equipment, which is the case for screen-printed electrodes and most microfabricated electrochemical biosensors, respectively, it is a highly attractive choice for electrochemical sensing (Figure 7).

Scanning electron micrographs of the top-view of the electrode (Figure 8A and 8B) display a highly structured graphene film inscribed onto the polyimide foil with a large surface area. The cross-sectional view of the LSG electrode (Figure 8C) gives access to the sheet-like structure of the graphene layers with an average thickness of 35 $\mu$m.

Raman spectroscopy was used to prove the quality of the laser-scribed 2D-nanomaterial. Figure 8D shows the presence of the typical D, G and 2D peaks. The D peak at 1352 cm$^{-1}$ suggests that there is a significant number of sp$^3$ centers present in the LSG due to structural edge defects. The prominent G band at 1584 cm$^{-1}$ and the sharp 2D band at 2702 cm$^{-1}$ further prove the presence of multi-layer graphene, as these bands are usually found at ~1580 cm$^{-1}$ and ~2700 cm$^{-1}$, respectively. In contrast, the 2D peak of bulk graphite consists of two components.[21] The electrochemically active surface and the electron transfer rate of the LSG electrodes was determined using cyclic voltammetry (Figure 10A). The electrochemically active graphene surface area determined by the Randles-Sevcik plot (Figure 10B) is 1.3 $\pm$ 0.2 cm$^2$, which is more than 18-fold the physical area (0.07 cm$^2$) and is due to the lamella-like structure of the LSG (Figures 8A-C). The electron transfer rate ($k^0$) of LSG in phosphate buffered saline (PBS, pH 7.4) containing 5 mM $K_3[Fe(CN)_6]$ was calculated with an extended method of the Nicholson treatment[22] to be $k^0 = 0.0044 \pm 0.0003$ cm$\cdot$s$^{-1}$. This exceeds the $k^0$ of other carbon materials such as edge-plane (0.002601 cm$\cdot$s$^{-1}$) or basal-plane pyrolytic graphite (0.00033 cm$\cdot$s$^{-1}$). Using more complicated fabrication procedures, the $k^0$ can even be further increased as shown by Griffiths *et al.* ($k^0 = 0.02373$ cm$\cdot$s$^{-1}$), but comes at the cost of an additional drop coating step with graphene oxide prior to laser-scribing.[5]

Initial studies of the modification protocol revealed that pristine LSG does not possess enough functional groups for adequate immobilization of anti-thrombin aptamer (Figure 11). For modification of the LSG electrodes with biorecognition molecules the graphene surface was modified with 1-pyrenebutyric acid via $\pi$-stacking interactions. This renders the electrode surface to bear a large number of COOH-groups available for rapid and gentle covalent coupling to amino-functionalized biorecognition elements such as the aptamer (Figure 7). The consecutive functionalization steps were monitored via differential pulse voltammetry and can be seen in Figure 12.

Starting with the bare LSG electrode, the peak current of the unmodified electrode is as high as 130 $\mu$A due to an unhindered diffusion of the redox marker hexacyanoferrate(II/III) to the electrode surface. Upon addition of the first layer consisting of 1-pyrenebutyric acid, the peak current decreases to 57.5 $\pm$ 0.2% of the original value of non-modified LSG. After coupling of the amino-functionalized anti-thrombin aptamers to the carboxyl groups, the peak current further decreases to 34.2 $\pm$ 0.4% indicating the successful modification of the laser-scribed graphene electrode with the biorecognition elements.

In order to quantify the thrombin sensing properties of such modified LSG electrodes, the device was immersed into PBS containing thrombin in varying concentrations from 1 to 100 pM. Equilibration time was found to be at an optimum at 30 min which is faster than comparable approaches using redox-tagged aptamers[23] or an impedimetric sensing scheme.[24] After rinsing the electrode with PBS, it was placed in the electrochemical cell for DPV measurements. As expected, upon binding of thrombin to the aptamers, the available electrode surface for the redox marker is reduced resulting in decreasing DPV peak currents upon increasing thrombin concentrations. Thrombin binding to the LSG electrodes shows a saturation behavior common for antigen-antibody or antigen-aptamer interactions. Using the logarithmic concentration scale (Figure 9A), the DPV peak currents decrease linearly following the equation $y = -33.9 -2.41x$, where y is the signal drop in $\mu A \cdot cm^{-2}$ and x is the logarithm of the thrombin concentration in $mol \cdot L^{-1}$ ($R^2=0.97$). Even a thrombin concentration as low as 1 $pmol \cdot L^{-1}$ results in a significant signal drop of $-5.2 \pm 0.3$ $\mu A \cdot cm^{-2}$ increasing to $-10.2 \pm 03.6$ $\mu A \cdot cm^{-2}$ at 100 pM thrombin.

With its low limit of detection (LOD) of 1 pM and its high sensitivity of $-2.41 \pm 0.16$ $\mu A \cdot cm^{-2}$ per logarithmic concentration unit, this sensor is particularly useful when measuring pathological thrombin concentrations below the nanomolar level as found in healthy patients.[23] In fact, it compares favorably to the performance of other carbon-based thrombin sensing schemes with higher LODs (Table 3).[24-26]

Furthermore, aptamer-functionalized LSG excels at electrode-to-electrode reproducibility. The largest variation of four individual electrodes is 2.2% of the mean signal change at a thrombin concentration of 10 pM.

The signal change of this sensing setup is based on the specific binding of thrombin to the immobilized aptamers and the resulting hindrance of diffusion of the redox marker. However, non-specific binding of interfering substances such as other proteins can also impede this diffusion and thus lead to a decrease in the DPV peak current. Therefore, interference studies were performed with buffer containing bovine serum albumin (BSA) at a very high concentration of 40 $g \cdot L^{-1}$ and also with serum. After immersing the electrode into the solution for 30 min and rinsing with PBS, the DPV peak current drops significantly due to non-specific adhesion of BSA and serum proteins, respectively. Using these as blank values for the measurements with thrombin-spiked buffer with BSA and serum, increasing concentrations of thrombin correlated to the expected decreasing DPV peak currents (Figure 9B). The lowest detectable thrombin concentration in both cases is as low as 5 $pmol \cdot L^{-1}$ which is similar to the LOD in pure buffer. Also, in comparison to the measurements in PBS, both calibration curves show no deterioration of the sensitivity. In contrast, sensitivity is slightly increasing to $-3.9 \pm 0,3$ $\mu A \cdot cm^{-2}$ per logarithmic concentration unit for the PBS with 40 $g \cdot L^{-1}$ BSA (Figure 11) due to BSA's stabilizing effects on thrombin and is then decreasing again to $-2.48 \pm 0.04$ $\mu A \cdot cm^{-2}$ per logarithmic concentration unit in presence of the numerous interfering substances present in serum (Figure 12). This shows the high robustness of aptamer-modified laser-scribed graphene as sensor material in biomolecule detection.

Figure 13 shows the change of the DPV peak current in phosphate buffered saline at pH 7.4 containing 5 mM $K_3[Fe(CN)_6]$ of 1-pyrenebutyric acid-modified LSG electrode after aptamer immobilization vs. Ag/AgCl at varying thrombin concentration in PBS containing 40 $g \cdot L^{-1}$ bovine serum albumin. n=3. Figure 14 shows the change of the DPV peak current in PBS at pH 7.4 containing 5 mM $K_3[Fe(CN)_6]$ of 1-pyrenebutyric acid-modified LSG electrode after aptamer immobilization vs. Ag/AgCl at varying thrombin concentration in serum. n=3.

In conclusion, we have demonstrated the outstanding capabilities of directly-printed LSG electrodes to serve as electrochemical transducer in complex bioanalytical sensing environments. Our approach offers a combination of simple and scalable electrode fabrication of high-quality graphene, and a universal surface functionalization route with aptamers using PBA due to the 3D interconnected self-supporting network of LSG. Any biorecognition element can easily be immobilized on its surface and quantitative and sensitive detection is possible even in the most complex matrices such as serum. We believe that LSG has the capability to advance clinical diagnostics through miniaturized all-carbon electrode microarrays, flexible, implantable diagnostic tools, and multiplexed analysis.

## EXPERIMENTAL SECTION

*Materials*: The polyimide foil for the LSG electrodes was purchased at Dasom RMS Co. LTD (Seoul, South Korea). 1-Pyrenebutyric acid, 1-ethyl-3-(3-dimethylaminopropyl)carbodiimide, N-hydroxysuccinimide, thrombin and fetal calf serum were obtained from Sigma-Aldrich (Taufkirchen, Germany). Anti-thrombin aptamer (5'-H2N-C6-GGTTGGTGTGGTTGG-3') was purchased from Eurofins Genomics (Ebersberg, Germany). All other chemicals were obtained from VWR International (Darmstadt, Germany) and were of analytical grade.

*Electrode Fabrication*: The working electrodes (3 mm diameter) were printed on an X-660 laser cutter platform (Universal Laser Systems, Scottsdale, AZ, USA) with a wavelength of 10.6 $\mu m$. The optimized laser power, speed, pulses per inch (PPI) and the Z-distance between laser and the sample were 0.81 W, 5.8 $cm \cdot s^{-1}$, 1000 PPI and 2 mm, respectively. All of the laser scribing experiments were performed under ambient conditions.

*Electrode Characterization*: The scanning electron microscope (SEM) images were recorded on a JSM-651 0 scanning electron microscope (JEOL GmbH, Freising, Germany) at 10 kV. Raman microscopy was performed on a DXR Raman microscope (Thermo Fisher Scientific GmbH, Dreieich, Germany) at 532 nm laser excitation (10 mW).

*Electrode Modification*: First, LSG electrodes were modified with 5 mM 1-pyrenebutyric acid in DMSO for 60 min. Further, the electrodes were immersed in an aqueous solution 50 mM EDC and 50 mM NHS for 90 min followed by 1 $\mu M$ anti-thrombin aptamer (5'-$H_2$N-C6-GGTTGGTGTGGTTGG-3') in an aqueous solution consisting of 100 mM NaCl and 4 mM EDTA for 3 h. The electrodes were extensively rinsed with phosphate buffered saline (PBS, pH 7.4).

*Electrochemical characterization.* This was performed using a CHI 650A Electrochemical Analyzer (CH Instruments, Austin, TX). A Pt wire counter electrode, Ag/AgCl reference electrode, and the respective working electrode were inserted into a 10 mL three-necked round-bottom flask and a solution volume of 7 mL was used for the measurements. The electrochemical measurements of the LSG electrode was performed in phosphate-buffered saline (PBS) containing 137 mM NaCl, 2.7 mM KCl, 10 mM $Na_2HPO_4$ and 1.8 mM $KH_2PO_4$ as well as 5 mM of the redox marker $K_3[Fe(CN)_6]$. The pH was adjusted to 7.4. Cyclic voltammograms were recorded from -0.3 V to 0.6 V with varying scan rates from 10 mV·s$^{-1}$ to 200 mV·s$^{-1}$.

*Electrochemical Thrombin Detection*: The aptamer-modified LSG electrode was immersed into PBS containing thrombin at concentrations from 1 to 100 pM for 30 min. After rinsing with PBS, DPV measurements were performed with a potential ranging from -0.3 and 0.6 V vs. Ag/AgCl with a pulse width of 0.2 s and an amplitude of 50 mV. For the interference studies, bovine serum albumin was solved in PBS in a concentration of 40 g·L$^{-1}$. The electrode was immersed into this solution containing thrombin at concentrations from 0 (blank) to 100 pM for 30 min. After rinsing with PBS, DPV measurements were again performed with a potential ranging from -0.3 and 0.6 V vs. Ag/AgCl with a pulse width of 0.2 s and an amplitude of 50 mV. As real samples, fetal calf serum was used. The electrode was immersed into the serum containing thrombin at concentrations from 0 (blank) to 1 00 pM for 30 min. After rinsing with PBS, DPV measurements were performed with the same adjustments as described above.

Table 3. Comparison of different thrombin detection schemes.

| Electrode material | Measurement method | Incubation time | Limit of detection | Interference studies | Reference |
|---|---|---|---|---|---|
| Reduced graphene oxide | Impedance spectroscopy | 60 min | 10 nM | In buffer | 24 |
| Pyrolyzed carbon | Impedance spectroscopy | 15 min | 0.5 nM | In buffer | 25 |
| Screen-printed carbon/ AuNPs[a] | Stripping voltammetry | 60 min | 1 nM | In buffer | 26 |
| Reduced graphene oxide | Differential pulse voltammetry | 20 min | 0.45 fM | None | 27 |
| Gold | Differential pulse voltammetry | 180 min | 6.4 nM | In serum | 23 |
| Laser-scribed graphene | Differential pulse voltammetry | 30 min | 1 pM | In buffer and serum | This work |

[a] Gold nanoparticles

## REFERENCES for Example 2

(1) Taleat, Z.; Khoshroo, A.; Mazloum-Ardakani, M. MicrochimActa 2014, 181 (9-10), 865-891.

(2) Ndamanisha, J. C.; Guo, L. Analytica Chimica Acta 2012, 747, 19-28.

(3) Gan, T.; Hu, S. Microchim Acta 2011, 175 (1-2), 1.

(4) McCreery, R. L.; McDermott, M. T. Anal. Chem. 2012, 84 (5), 2602-2605.

(5) Griffiths, K.; Dale, C.; Hedley, J.; Kowal, M. D.; Kaner, R. B.; Keegan, N. Nanoscale 2014, 6 (22), 13613-13622.

(6) Shao, Y.; Wang, J.; Wu, H.; Liu, J.; Aksay, I. A.; Lin, Y. Electroanalysis 2010, 22 (10), 1027-1036.

(7) Eigler, S.; Hirsch, A. Angew. Chem. Int. Ed. 2014, 53 (30), 7720-7738.

(8) Zöpfl, A.; Sisakthi, M.; Eroms, J.; Matysik, F.-M.; Strunk, C.; Hirsch, T. Microchim Acta 2015, 183 (1), 83-90.

(9) Kang, X.; Wang, J.; Wu, H.; Aksay, I. A.; Liu, J.; Lin, Y. Biosensors and Bioelectronics 2009, 25 (4), 901-905.

(10) Wang, X.; Kholmanov. I.; Chou, H.; Ruoff, R. S. ACS Nano 2015, 9 (9), 8737-8743.

(11) Ratinac, K. R.; Yang, W.; Gooding, J. J.; Thordarson, P.; Braet, F. Electroanalysis 2011, 23 (4), 803-826.

(12) Ito, Y.; Tanabe, Y.; Qiu, H.-J.; Sugawara, K.; Heguri, S.; Tu, N. H.; Huynh, K. K.; Fujita, T.; Takahashi, T.; Tanigaki, K.; Chen, M. Angew. Chem. Int. Ed. 2014, 53 (19), 4822-4826.

(13) El-Kady, M. F.; Strong, V.; Dubin, S.; Kaner, R. B. Science 2012, 335 (6074), 1326-1330.

(14) Strong, V.; Dubin, S.; El-Kady, M. F.; Lech, A.; Wang, Y.; Weiller, B. H.; Kaner, R. B. ACS Nano 2012, 6 (2), 1395-1403.

(15) Tian, H.; Shu, Y.; Cui, Y.-L.; Mi, W.-T.; Yang, Y.; Xie, D.; Ren, T.-L. Nanoscale 2014, 6 (2), 699-705.

(16) Lin, J.; Peng, Z.; Liu, Y.; Ruiz-Zepeda, F.; Ye, R.; Samuel, E. L. G.; Yacaman, M. J.; Yakobson, B. I.; Tour, J. M. Nature Communications 2014, 5, 5714.

(17) Nayak, P.; Kurra, N.; Xia, C.; Alshareef, H. N. Adv. Electron. Mater. 2016, 2 (10).

(18) Sanghavi, B. J.; Wolfbeis, O. S.; Hirsch, T.; Swami, N. S. Microchim Acta 2014, 182 (1-2), 1-41.

(19) Gupta, R. K.; Pandya, R.; Sieffert, T.; Meyyappan, M.; Koehne, J. E. Journal of Electroanalytical Chemistry 2016, 773, 53-62.

(20) Criado, A.; Melchionna, M.; Marchesan, S.; Prato, M. Angew. Chem. Int. Ed. 2015, 54 (37), 10734-10750.

(21) Ferrari, A. C. Solid State Communications 2007, 143 (1-2), 47-57.

(22) Lavagnini, I.; Antiochia, R.; Magno, F. Electroanalysis 2004, 16 (6), 505-506.

(23) Xiao, Y.; Lubin, A. A.; Heeger, A. J.; Plaxco, K. W. Angewandte Chemie 2005, 117 (34), 5592-5595.

(24) Loo, A. H.; Bonanni, A.; Pumera, M. Nanoscale 2012, 4 (1), 143-147.

(25) Lee, J. A.; Hwang, S.; Kwak, J.; Park, S. I.; Lee, S. S.; Lee, K.-C. Sensors and Actuators B: Chemical 2008, 129 (1), 372-379.

(26) Suprun, E.; Shumyantseva, V.; Bulko, T.; Rachmetova, S.; Rad'ko, S.; Bodoev, N.; Archakov, A. Biosensors and Bioelectronics 2008, 24 (4), 825-830.

(27) Wang, Y.; Xiao, Y.; Ma, X.; Li, N.; Yang, X. Chem Commun 2012, 48 (5), 738-740.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1 % to about 5%" should be interpreted to include not only the explicitly recited concentration of about 0.1 wt% to about 5 wt%, but also include individual concentrations (*e.g.*, 1 %, 2%, 3%, and 4%) and the sub-ranges (*e.g.,* 0.5%, 1.1%, 2.2%, 3.3%, and 4.4%) within the indicated range. In an embodiment, the term "about" can include traditional rounding according to significant figures of the numerical value. In addition, the phrase "about 'x' to 'y'" includes "about 'x' to about 'y'".

**Claims**

1. A biosensing device, comprising:

   an on-chip electrode platform disposed on a polymer substrate, comprising:

   a three-dimensional laser scribed graphene counter electrode;
   a three-dimensional laser scribed graphene working electrode; and
   a three-dimensional laser scribed graphene electrode, the electrodes being directly grown on the substrate, and wherein the three-dimensional laser scribed graphene counter electrode, working electrode, and electrode have a self-standing macro-mesoporous three-dimensional morphology comprising a macroporous surface having a mesoporous network of pores, **characterized in that**
   the three-dimensional laser scribed graphene working electrode includes Pt nanoparticles disposed on the three-dimensional laser scribed graphene surface, and
   the three-dimensional laser scribed graphene working electrode includes 1-pyrenbutyric acid anchored to graphene of the three-dimensional laser scribed graphene working electrode and a bioreceptor covalently attached to a carboxyl group of the 1-pyrenbutyric acid.

2. The device of claim 1, wherein the bioreceptor is an aptamer.

3. The device of claim 1, wherein the polymer substrate is polyimide.

4. A method of making an on-chip electrode platform for biosensing, comprising:

   forming electrodes of the on-chip electrode platform on a polyimide substrate by directing a laser beam onto the polyimide substrate, wherein the electrodes comprise a three-dimensional laser scribed graphene counter electrode, a three-dimensional laser scribed graphene working electrode, and a three-dimensional laser scribed graphene electrode, wherein the three-dimensional laser scribed graphene counter electrode, working electrode, and electrode have a self-standing macro/mesoporous three-dimensional morphology comprising a macroporous surface having a mesoporous network of pores;
   disposing Pt nanoparticles on the three-dimensional laser scribed graphene working electrode;
   anchoring 1-pyrenbutyric acid to graphene of the three-dimensional laser scribed graphene working electrode; and

covalently attaching a bioreceptor to a carboxyl group of the 1-pyrenebutyric acid.

5. The method of claim 4, wherein the bioreceptor is an aptamer.

6. The method of claim 4, wherein the laser beam produces a local temperature on the substrate of about 2500 °C or more.

7. The method of claim 4, wherein the three-dimensional laser scribed graphene counter electrode, working electrode, and electrode have a thickness of 10 $\mu$m to 50 $\mu$m.

8. The method of claim 4, wherein the Pt nanoparticles are disposed using electrodeposition.

9. The method of claim 4, further comprising:
defining, by selective passivation, the working electrode area on the polyimide substrate by applying a PDMS coating on the polyimide substrate.

**Patentansprüche**

1. Biosensing-Vorrichtung, umfassend:
eine On-Chip-Elektrodenplattform, die auf einem Polymersubstrat angeordnet ist, umfassend:

eine dreidimensionale lasergeschriebene Graphen-Gegenelektrode;
eine dreidimensionale lasergeschriebene Graphen-Arbeitselektrode; und
eine dreidimensionale lasergeschriebene Graphenelektrode, wobei die Elektroden direkt auf dem Substrat gezüchtet werden, und
wobei die dreidimensionale lasergeschriebene Graphen-Gegenelektrode, Arbeitselektrode und Elektrode eine eigenständige makromesoporöse, dreidimensionale Morphologie aufweisen, umfassend eine makroporöse Oberfläche mit einem mesoporösen Netz von Poren, **dadurch gekennzeichnet, dass**
die dreidimensionale lasergeschriebene Graphen-Arbeitselektrode Pt-Nanopartikel einschließt, die auf der dreidimensionalen lasergeschriebenen Graphenoberfläche angeordnet sind, und
die dreidimensionale lasergeschriebene Graphen-Arbeitselektrode 1-Pyrenbuttersäure einschließt, die mit Graphen der dreidimensionalen lasergeschriebenen Graphen-Arbeitselektrode verankert ist, und einen Biorezeptor, der kovalent an ein Carboxylgruppe der 1-Pyrenbuttersäure befestigt ist.

2. Vorrichtung nach Anspruch 1, wobei der Biorezeptor ein Aptamer ist.

3. Vorrichtung nach Anspruch 1, wobei das Polymersubstrat Polyimid ist.

4. Verfahren zum Herstellen einer On-Chip-Elektrodenplattform zum Biosensing, umfassend:

Bilden von Elektroden der On-Chip-Elektrodenplattform auf einem Polyimidsubstrat durch Ausrichten eines Laserstrahls auf das Polyimidsubstrat, wobei die Elektroden eine dreidimensionale lasergeschriebene Graphen-Gegenelektrode, eine dreidimensionale lasergeschriebene Graphen-Arbeitselektrode und eine dreidimensionale lasergeschriebene Graphenelektrode umfassen, wobei die dreidimensionale lasergeschriebene Graphen-Gegenelektrode, Arbeitselektrode und Elektrode eine eigenständige makro-/mesoporöse dreidimensionale Morphologie aufweisen, umfassend eine makroporöse Oberfläche mit einem mesoporösen Netz von Poren,
Anordnen von Pt-Nanopartikeln auf der dreidimensionalen lasergeschriebene Graphenoberfläche;
Verankern von 1-Pyrenbuttersäure auf Graphen der dreidimensionalen lasergeschriebenen Graphen-Arbeitselektrode, und
kovalentes Befestigen eines Biorezeptors an einer Carboxylgruppe der 1-Pyrenbuttersäure.

5. Verfahren nach Anspruch 4, wobei der Biorezeptor ein Aptamer ist.

6. Verfahren nach Anspruch 4, wobei der Laserstrahl eine lokale Temperatur auf dem Substrat von ungefähr 2500 °C oder mehr erzeugt.

7. Verfahren nach Anspruch 4, wobei die dreidimensionale lasergeschriebene Graphen-Gegenelektrode, Arbeitselektrode und Elektrode eine Dicke von 10 μm bis 50 μm aufweisen.

**8.** Verfahren nach Anspruch 4, wobei die Pt-Nanopartikel unter Verwendung von Elektroabscheidung angeordnet sind.

**9.** Verfahren nach Anspruch 4, weiter umfassend
Definieren, durch selektive Passivierung, des Arbeitselektrodenbereichs auf dem Polyimidsubstrat durch Anwenden einer PDMS-Beschichtung auf das Polyimidsubstrat.

**Revendications**

**1.** Dispositif de biodétection, comprenant :
une plateforme d'électrodes sur puce disposée sur un substrat de polymère, comprenant :

une contre-électrode de graphène inscrit au laser tridimensionnel ;
une électrode de travail de graphène inscrit au laser tridimensionnel ; et
une électrode de graphène inscrit au laser tridimensionnel, les électrodes étant formées directement sur le substrat, et
dans lequel la contre-électrode, l'électrode de travail, et l'électrode de graphène inscrit au laser tridimensionnel ont une morphologie tridimensionnelle macro-mésoporeuse autoportante comprenant une surface macroporeuse ayant un réseau de pores mésoporeux, **caractérisé en ce que**
l'électrode de travail de graphène inscrit au laser tridimensionnel comprend des nanoparticules de Pt disposées sur la surface du graphène inscrit au laser tridimensionnel, et
l'électrode de travail de graphène inscrit au laser tridimensionnel comprend de l'acide 1-pyrènebutyrique ancré au graphène de l'électrode de travail de graphène inscrit au laser tridimensionnel et un biorécepteur lié de façon covalente à un groupe carboxyle de l'acide 1-pyrènebutyrique.

**2.** Dispositif selon la revendication 1, dans lequel le biorécepteur est un aptamère.

**3.** Dispositif selon la revendication 1, dans lequel le substrat de polymère est du polyimide.

**4.** Procédé de fabrication d'une plateforme d'électrodes sur puce pour biodétection, comprenant :

la formation d'électrodes de la plateforme d'électrodes sur puce sur un substrat de polyimide par orientation d'un faisceau laser sur le substrat de polyimide, dans lequel les électrodes comprennent une contre-électrode de graphène inscrit au laser tridimensionnel, une électrode de travail de graphène inscrit au laser tridimensionnel et une électrode de graphène inscrit au laser tridimensionnel, dans lequel la contre-électrode, l'électrode de travail et l'électrode de graphène inscrit au laser tridimensionnel ont une morphologie tridimensionnelle macro/mésoporeuse autoportante comprenant une surface macroporeuse ayant un réseau de pores mésoporeux ;
la disposition de nanoparticules de Pt sur l'électrode de travail de graphène inscrit au laser tridimensionnel ;
l'ancrage d'acide 1-pyrènebutyrique au graphène de l'électrode de travail de graphène inscrit au laser tridimensionnel ; et
la liaison covalente d'un biorécepteur à un groupe carboxyle de l'acide 1-pyrènebutyrique.

**5.** Procédé selon la revendication 4, dans lequel le biorécepteur est un aptamère.

**6.** Procédé selon la revendication 4, dans lequel le faisceau laser produit une température locale sur le substrat d'environ 2500 °C ou plus.

**7.** Procédé selon la revendication 4, dans lequel la contre-électrode, l'électrode de travail et l'électrode de graphène inscrit au laser tridimensionnel ont une épaisseur de 10 μm à 50 μm.

**8.** Procédé selon la revendication 4, dans lequel les nanoparticules de Pt sont disposées par électrodéposition.

**9.** Procédé selon la revendication 4, comprenant en outre :
la définition, par passivation sélective, de la zone d'électrode de travail sur le substrat de polyimide par application d'un revêtement de PDMS sur le substrat de polyimide.

Fig. 1A

Fig. 1B

Fig. 1C

PI/LSG

LSG

Fig. 1E

Fig. 1D

Fig. 2A  Fig. 2B

Fig. 2C  Fig. 2D

Fig. 2E

Fig. 2F

Fig. 2G

Fig. 2H

Fig. 3A

Fig. 3B

Fig. 3C

Fig. 3D

Fig. 4A    Fig. 4B    Fig. 4C

Fig. 4D    Fig. 4E    Fig. 4F

Fig. 5

Fig. 6

**Electrode fabrication**

Laser patterning → Surface passivation → Laser-scribed graphene electrode

**Electrode functionalization**

5 mM 1-pyrenebutyric acid

1 h

Unmodified LSG electrode

Carboxy-functionalized LSG electrode

1) 50 mM EDC + 50 mM NHS, 90 min

2) 5 µM Aptamer, 3 h

Aptamer-functionalized LSG electrode

Fig. 7

Fig. 8A

Fig. 8B

Fig. 8C

Fig. 8D

Fig. 9A

Fig. 9B

Fig. 10A

Fig. 10B

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62364917
- JP 2016045032 A

### Non-patent literature cited in the description

- **LUO SIDA et al.** *Direct laser writing for creating porous graphitic structures and their use for flexible and highly sensitive sensor and sensor arrays*
- **KATIE GRIFFITHS et al.** *Laser-scribed graphene presents an opportunity to print a new generation of disposable electrochemical sensors*
- **VERONICA STRONG et al.** *Patterning and Electronic Tuning of Laser Scribed Graphene for Flexible All Carbon Devices*
- **TALEAT, Z. ; KHOSHROO, A. ; MAZLOUM-AR-DAKANI, M.** *MicrochimActa,* 2014, vol. 181 (9-10), 865-891
- **NDAMANISHA, J. C. ; GUO, L.** *Analytica Chimica Acta,* 2012, vol. 747, 19-28
- **GAN, T. ; HU, S.** *Microchim Acta,* 2011, vol. 175 (1-2), 1
- **MCCREERY, R. L. ; MCDERMOTT, M. T.** *Anal. Chem.,* 2012, vol. 84 (5), 2602-2605
- **GRIFFITHS, K. ; DALE, C. ; HEDLEY, J. ; KOWAL, M. D. ; KANER, R. B. ; KEEGAN, N.** *Nanoscale,* 2014, vol. 6 (22), 13613-13622
- **SHAO, Y. ; WANG, J. ; WU, H. ; LIU, J. ; AKSAY, I. A. ; LIN, Y.** *Electroanalysis,* 2010, vol. 22 (10), 1027-1036
- **EIGLER, S. ; HIRSCH, A.** *Angew. Chem. Int. Ed.,* 2014, vol. 53 (30), 7720-7738
- **ZÖPFL, A ; SISAKTHI, M. ; EROMS, J. ; MATYSIK, F.-M. ; STRUNK, C. ; HIRSCH, T.** *Microchim Acta,* 2015, vol. 183 (1), 83-90
- **KANG, X. ; WANG, J. ; WU, H. ; AKSAY, I. A. ; LIU, J. ; LIN, Y.** *Biosensors and Bioelectronics,* 2009, vol. 25 (4), 901-905
- **WANG, X. ; KHOLMANOV. I. ; CHOU, H. ; RUOFF, R. S.** *ACS Nano,* 2015, vol. 9 (9), 8737-8743
- **RATINAC, K. R. ; YANG, W. ; GOODING, J. J. ; THORDARSON, P. ; BRAET, F.** *Electroanalysis,* 2011, vol. 23 (4), 803-826
- **ITO, Y. ; TANABE, Y. ; QIU, H.-J. ; SUGAWARA, K. ; HEGURI, S. ; TU, N. H. ; HUYNH, K. K. ; FUJITA, T. ; TAKAHASHI, T. ; TANIGAKI, K.** *Angew. Chem. Int. Ed.,* 2014, vol. 53 (19), 4822-4826
- **EL-KADY, M. F. ; STRONG, V. ; DUBIN, S. ; KANER, R. B.** *Science,* 2012, vol. 335 (6074), 1326-1330
- **STRONG, V. ; DUBIN, S. ; EL-KADY, M. F. ; LECH, A. ; WANG, Y. ; WEILLER, B. H. ; KANER, R. B.** *ACS Nano,* 2012, vol. 6 (2), 1395-1403
- **TIAN, H. ; SHU, Y. ; CUI, Y.-L. ; MI, W.-T. ; YANG, Y. ; XIE, D. ; REN, T.-L.** *Nanoscale,* 2014, vol. 6 (2), 699-705
- **LIN, J. ; PENG, Z. ; LIU, Y. ; RUIZ-ZEPEDA, F. ; YE, R. ; SAMUEL, E. L. G. ; YACAMAN, M. J. ; YAKOBSON, B. I. ; TOUR, J. M.** *Nature Communications,* 2014, vol. 5, 5714
- **NAYAK, P. ; KURRA, N. ; XIA, C. ; ALSHAREEF, H. N.** *Adv. Electron. Mater.,* 2016, vol. 2 (10
- **SANGHAVI, B. J. ; WOLFBEIS, O. S. ; HIRSCH, T. ; SWAMI, N. S.** *Microchim Acta,* 2014, vol. 182 (1-2), 1-41
- **GUPTA, R. K. ; PANDYA, R. ; SIEFFERT, T. ; MEYYAPPAN, M. ; KOEHNE, J. E.** *Journal of Electroanalytical Chemistry,* 2016, vol. 773, 53-62
- **CRIADO, A. ; MELCHIONNA, M. ; MARCHESAN, S. ; PRATO, M.** *Angew. Chem. Int. Ed.,* 2015, vol. 54 (37), 10734-10750
- **FERRARI, A. C.** *Solid State Communications,* 2007, vol. 143 (1-2), 47-57
- **LAVAGNINI, I. ; ANTIOCHIA, R. ; MAGNO, F.** *Electroanalysis,* 2004, vol. 16 (6), 505-506
- **XIAO, Y. ; LUBIN, A. A. ; HEEGER, A. J. ; PLAXCO, K. W.** *Angewandte Chemie,* 2005, vol. 117 (34), 5592-5595
- **LOO, A. H. ; BONANNI, A. ; PUMERA, M.** *Nanoscale,* 2012, vol. 4 (1), 143-147
- **LEE, J. A. ; HWANG, S. ; KWAK, J. ; PARK, S. I. ; LEE, S. S. ; LEE, K.-C.** *Sensors and Actuators B: Chemical,* 2008, vol. 129 (1), 372-379
- **SUPRUN, E. ; SHUMYANTSEVA, V. ; BULKO, T. ; RACHMETOVA, S. ; RAD'KO, S. ; BODOEV, N. ; ARCHAKOV, A.** *Biosensors and Bioelectronics,* 2008, vol. 24 (4), 825-830
- **WANG, Y. ; XIAO, Y. ; MA, X. ; LI, N. ; YANG, X.** *Chem Commun,* 2012, vol. 48 (5), 738-740